# EUROPÄISCHE PATENTANMELDUNG

(11) **EP 4 505 970 A1**
(43) Veröffentlichungstag der Anmeldung: **12.02.2025**
(21) Anmeldenummer: 24192433.1
(22) Anmeldetag: 01.08.2024
(51) Int. Cl.: A61B 90/70, A61L 2/18, B08B 3/02, A61B 1/12

(54) **REINIGUNGSVORRICHTUNG**

(30) Priorität: 08.08.2023 DE 102023121049
(71) Anmelder: MMM Münchener Medizin Mechanik GmbH, 82152 Planegg (DE)
(72) Erfinder: TSCHUI, Stefan, 86971 Peiting (DE); FÜRG, Markus, 86684 Holzheim (DE); NÄGELE, Tobias, 87640 Biessenhofen (DE)
(74) Vertreter: Wittmann, Ernst-Ulrich

(57) **Zusammenfassung**

Eine Reinigungsvorrichtung zum Reinigen und Desinfizieren von wiederaufbereitbaren Medizinprodukten wird bereitgestellt. Die Reinigungsvorrichtung umfasst eine Reinigungskammer, die mindestens eine verschließbare Öffnung zum Be- und/oder Entladen der Reinigungskammer aufweist, und einen Anschlussarm, der in der Reinigungskammer vorgesehen ist. Der Anschlussarm hat eine Anschlussfläche mit mindestens einem Reinigungsfluidanschluss, der mit einer Reinigungsfluidzuführungseinrichtung der Reinigungsvorrichtung in Verbindung steht. Die Anschlussfläche ist mit einem Anschlussadapter, der mindestens eine Reinigungsfluidleitung aufweist, verbindbar, so dass der Reinigungsfluidanschluss mit der Reinigungsfluidleitung in Verbindung steht, um Reinigungsfluid von der Reinigungsfluidzuführungseinrichtung einem Innenkanal des Medizinprodukts zur Innenreinigung zuzuführen. Die Anschlussfläche ist bezüglich einer Innenwand der Reinigungskammer in Richtung der Öffnung der Reinigungskammer geneigt.

## Beschreibung

### Technisches Gebiet

Die vorliegende Erfindung betrifft eine Reinigungsvorrichtung zum Reinigen und Desinfizieren von wiederaufbereitbaren Medizinprodukten, insbesondere Endoskopen.

### Stand der Technik

Wiederaufbereitbare Medizinprodukte müssen nach dem Gebrauch, z.B. bei einer Untersuchung oder einer Operation, für eine Wiederverwendung aufbereitet werden. Ein wichtiger Schritt zur Aufbereitung der Medizinprodukte, wie zum Beispiel Endoskope, chirurgische Instrumente, usw. ist die Reinigung und Desinfektion. Dieser Aufbereitungsschritt muss nach geprüften, validierten Verfahren und in automatisch arbeitenden Reinigungsvorrichtungen erfolgen, die definierte Leistungskriterien erfüllen.

Bei den Reinigungsvorrichtungen handelt es sich um Reinigungs- und Desinfektionsgeräte, deren allgemeine Spezifikationen und Leistungsanforderungen in harmonisierten Normen festgelegt sind (z. B. Normenreihe DIN EN ISO 15883).

Eine wichtige Funktion der Reinigungsvorrichtungen ist die Abreinigung der Produktoberflächen von Rückständen, z.B. Blut. Zu diesem Zweck werden die Produkte üblicherweise in Siebschalen (Trays) gelegt und über einen längeren Zeitraum in einer Reinigungskammer der Reinigungsvorrichtung mit einem Reinigungsfluid besprüht. Der Ablauf des Reinigungsprozesses ist im Allgemeinen mehrstufig und erfolgt unter Verwendung von unterschiedlichen Reinigungsfluiden und Temperaturen. Verwendung finden hier beispielsweise Wasser, Weichwasser mit Reinigungschemie versetzt und demineralisiertes Wasser. Zur Reduzierung des Wasserverbrauchs wird das verwendete Wasser im Kreislauf für eine definierte Zeit umgewälzt und wieder verworfen. Am Ende des Reinigungsprozesses erfolgt ein letzter Schritt der Spülung mit demineralisiertem Wasser.

Für die Außenreinigung der Medizinprodukte werden Sprüharme zur Verteilung des Reinigungsfluids über die zu reinigenden Medizinprodukte eingesetzt. Die Sprüharme sind in der Reinigungsvorrichtung oben und unten in der Reinigungskammer angeordnet und bei einer Beladung unter Verwendung von mehreren Siebschalen auf verschiedenen Ebenen zusätzlich zwischen den einzelnen Ebenen angeordnet. Die Sprüharme rotieren beim Versprühen des Reinigungsfluids innerhalb einer horizontalen Ebene, wodurch sichergestellt wird, dass das Reinigungsfluid an alle zu reinigende Stellen innerhalb der Reinigungskammer gelangt.

Je nach Beschaffenheit der wiederaufbereitbaren Medizinprodukte kann auch eine Innenreinigung von in den Medizinprodukten enthaltenen Innenräumen (z.B. Innenkanäle), die während einer Verwendung kontaminiert werden, erforderlich sein. Beispielsweise muss bei der Aufbereitung von Endoskopen in der Reinigungsvorrichtung jeder einzelne Innenkanal des Endoskops zur Reinigung mit einem definierten Durchfluss von Reinigungsfluid durchspült werden. Dazu werden die einzelnen Innenkanäle der Endoskope jeweils mittels spezieller Konnektoren mit Schläuchen (Reinigungsfluidleitungen) verbunden. Die einzelnen Schläuche werden dann in einem Anschlussadapter zusammengefasst.

In der Reinigungskammer ist eine Anschlussfläche vorgesehen, die mehrere Reinigungsfluidanschlüsse der Reinigungsvorrichtung aufweist. Die Anschlussfläche mit den Reinigungsfluidanschlüssen dient als Gegenstück für den Anschlussadapter. Durch ein Zusammenschließen des Anschlussadapters und der Anschlussfläche werden die einzelnen Schläuche, die mit den einzelnen Innenkanälen des Endoskops in Verbindung stehen, mit den Reinigungsfluidanschlüssen verbunden. Der Anschlussadapter und die Anschlussfläche sind im Allgemeinen so ausgeführt, dass jedem Innenkanal des Endoskops ein jeweiliger Reinigungsfluidanschluss der Reinigungsvorrichtung zugeordnet ist. Die Reinigungsfluidanschlüsse stehen mit einer Umwälzpumpe (Reinigungsfluidzuführungseinrichtung) der Reinigungsvorrichtung in Verbindung. Die Umwälzpumpe fördert ein Reinigungsfluid über die in der Anschlussfläche vorgesehenen Reinigungsfluidanschlüsse und die einzelnen Schläuche zu den einzelnen Innenkanälen des Endoskops.

Herkömmlicherweise ist die Anschlussfläche mit den Reinigungsfluidanschlüssen der Reinigungsvorrichtung an einer Seitenwand der Reinigungskammer angeordnet. In der Vergangenheit wurde die Anschlussfläche in der Nähe der Beladeseite der Reinigungskammer angeordnet, um eine leichte Handhabung beim Zusammenschließen des Anschlussadapters und der Anschlussfläche zu gewährleisten. Jedoch stellt die Positionierung der Anschlussfläche in der Nähe der Beladeseite bei modernen Reinigungsvorrichtungen keine Option mehr dar, da diese nach den Vorgaben gängiger Hygienekonzepte als zweitürige Reinigungsvorrichtungen mit einer Be- und Entladeseite ausgeführt werden. Dies erfolgt zur Sicherstellung der Trennung der sogenannten "unreinen Seite" (noch nicht aufbereitete Medizinprodukte) von der sogenannten "reinen Seite" (gereinigte und desinfizierte Medizinprodukte).

Bei zweitürigen Geräten wird daher die Anschlussfläche in der Regel in der Mitte der Seitenwand zwischen der Be- und Entladeseite angeordnet, um eine Zugänglichkeit von beiden Seiten zu ermöglichen. Die Zugänglichkeit ist jedoch durch die Positionierung in der Mitte der Reinigungskammer erschwert. Ein Bedienen der Reinigungsvorrichtung ist somit unter ergonomischen Gesichtspunkten nicht optimal. Zudem erfordert ein Zusammenschließen des Anschlussadapters und der Anschlussfläche von einem Benutzer ein verhältnismäßig tiefes Hineingreifen in die Reinigungskammer, wodurch die Gefahr einer Kontaminierung des Benutzers erhöht ist. Des Weiteren ist die Einsehbarkeit der Anschlussfläche stark eingeschränkt, wodurch ein Zusammenschließen des Anschlussadapters und der Anschlussfläche erschwert wird und fast ausschließlich durch Erfühlen der richtigen Anschlussposition möglich ist.

Somit besteht ein Bedarf nach einer Reinigungsvorrichtung, die eine verbesserte Bedienbarkeit aufweist. Des Weiteren besteht ein Bedarf nach einer Reinigungsvorrichtung, bei der die Gefahr einer Kontamination eines Benutzers geringer ist.

### Von der Erfindung zu lösende technische Aufgabe

Der vorliegenden Erfindung liegt daher die Aufgabe zugrunde, eine Reinigungsvorrichtung zum Reinigen und Desinfizieren von wiederaufbereitbaren Medizinprodukten, insbesondere Endoskopen, bereitzustellen, die eine verbesserte Bedienbarkeit bietet und die Gefahr einer Kontamination eines Benutzers verringert.

### Offenbarung der Erfindung

Die Aufgabe wird erfindungsgemäß mit einer Reinigungsvorrichtung gelöst, die die Merkmale des unabhängigen Anspruchs 1 aufweist. Weitere vorteilhafte Ausgestaltungen sind in den abhängigen Ansprüchen definiert.

Die erfindungsgemäße Reinigungsvorrichtung umfasst eine Reinigungskammer, die mindestens eine verschließbare Öffnung zum Be- und/oder Entladen der Reinigungskammer aufweist, und einen Anschlussarm, der in der Reinigungskammer vorgesehen ist. Der Anschlussarm hat eine Anschlussfläche mit mindestens einem Reinigungsfluidanschluss. Der Reinigungsfluidanschluss steht mit einer Reinigungsfluidzuführungseinrichtung der Reinigungsvorrichtung in Verbindung. Die Anschlussfläche ist eingerichtet, um mit einem Anschlussadapter, der mindestens eine Reinigungsfluidleitung aufweist, verbindbar zu sein, so dass der Reinigungsfluidanschluss mit der Reinigungsfluidleitung in Verbindung steht, um Reinigungsfluid von der Reinigungsfluidzuführungseinrichtung einem Innenkanal des Medizinprodukts zur Innenreinigung zuzuführen. Die Anschlussfläche ist bezüglich einer Innenwand der Reinigungskammer in Richtung der Öffnung der Reinigungskammer geneigt.

Durch die Neigung der Anschlussfläche in Richtung der Öffnung ist die Anschlussfläche für einen Benutzer leichter erreichbar und besser einsehbar. Dementsprechend bietet die erfindungsgemäße Reinigungsvorrichtung eine bessere Ergonomie, insbesondere auch eine bessere optische Ergonomie. Somit kann ein Benutzer das Zusammenschließen des Anschlussadapters und der Anschlussfläche leichter und zuverlässiger durchführen.

Die Reinigungskammer kann eine erste verschließbare Öffnung zum Beladen und eine der ersten Öffnung entgegengesetzte zweite verschließbare Öffnung zum Entladen aufweisen. Der Anschlussarm kann schwenkbar in der Reinigungskammer vorgesehen sein, sodass der Anschlussarm zwischen einer ersten Position, in der die Anschlussfläche bezüglich der Innenwand in Richtung der ersten Öffnung geneigt ist, und einer zweiten Position schwenkbar ist, in der die Anschlussfläche bezüglich der Innenwand in Richtung der zweiten Öffnung geneigt ist.

Durch den schwenkbar ausgeführten Anschlussarm kann bei einer zweitürigen Ausführung der Reinigungsvorrichtung sowohl das Verbinden des Anschlussadapters und der Anschlussfläche beim Beladen der Reinigungsvorrichtung beispielsweise von der "unreinen Seite" als auch das Trennen des Anschlussadapters von der Anschlussfläche beim Entladen der Reinigungsvorrichtung beispielsweise von der "reinen Seite" ergonomischer gestaltet werden.

Der Anschlussarm kann einen ersten Abschnitt, der sich in einer ersten Richtung erstreckt und an der Innenwand der Reinigungskammer gestützt ist, und einen zweiten Abschnitt umfassen, der sich in einer zweiten Richtung erstreckt, die bezüglich der ersten Richtung geneigt ist. Die Anschlussfläche kann an dem zweiten Abschnitt vorgesehen sein.

Durch die Ausbildung des Anschlussarms mit dem ersten Abschnitt und dem zweiten Abschnitt, der bezüglich des ersten Abschnitts geneigt ist, kann die Anschlussfläche innerhalb der Reinigungskammer weiter in Richtung Öffnung positioniert werden. Dadurch kann das Verbinden und ggf. das Trennen von Anschlussadapter und Anschlussfläche noch ergonomischer gestaltet werden. Insbesondere kann dadurch erreicht werden, dass ein Benutzer weniger weit in den Innenraum der Reinigungskammer hineingreifen muss, um die Anschlussfläche zu erreichen. Dies verringert die Gefahr einer Kontamination des Benutzers durch unreine Medizinprodukte. Außerdem können durch die Verbesserte Ergonomie beim Verbinden und Trennen des Anschlussadapters und der Anschlussfläche Beschädigungen am Medizinprodukt und insbesondere an den Reinigungsfluidleitungen verhindert werden.

Der erste Abschnitt kann an der Innenwand der Reinigungskammer um die erste Richtung herum schwenkbar gestützt sein.

Die zweite Richtung und die Anschlussfläche können zueinander in einem Winkel von 25° bis 90° stehen. Insbesondere kann die Anschlussfläche rechtwinklig zu der zweiten Richtung sein.

Durch die Wahl des Winkels zwischen der Anschlussfläche und der zweiten Richtung im angegebenen Bereich kann eine anwendungsspezifische Ausrichtung der Anschlussfläche erfolgen.

Der Anschlussarm kann um 90° bis 170°, vorzugsweise um 120° bis 140°, schwenkbar gestützt sein.

Durch die Wahl des Schwenkwinkels im angegebenen Bereich kann ein besonders ergonomisches Verbinden bzw. Trennen des Anschlussadapters und der Anschlussfläche gewährleistet werden, wobei die Gefahr eines Verdrehens der Reinigungsfluidleitungen verhindert werden kann.

Der Schwenkbereich des Anschlussarms kann bezüglich einer Ebene, die parallel zu der ersten und der zweiten Öffnung verläuft, symmetrisch vorgesehen sein. Zudem kann der erste Abschnitt an der Innenwand im Wesentlichen mittig zwischen der ersten Öffnung und der zweiten Öffnung vorgesehen sein.

Dadurch kann die Ergonomie beim Verbinden und Trennen des Anschlussadapters und der Anschlussfläche sowohl beim Be- als auch beim Entladen in gleicher Weise verbessert werden.

Die Reinigungskammer kann eine obere Innenwand, eine untere Innenwand, sowie eine erste und eine zweite Seiteninnenwand aufweisen. Der erste Abschnitt kann an einer von der ersten und der zweiten Seiteninnenwand gestützt sein und sich parallel zu der oberen Innenwand sowie senkrecht zu der Seitenwand erstreckt.

Diese Ausgestaltung ist insbesondere für im Wesentlichen rechteckig ausgeführte Reinigungskammern vorteilhaft.

Die zweite Richtung, in die sich der zweite Abschnitt erstreckt, und die erste Richtung, in die sich der erste Abschnitt erstreckt, können in einem Winkel von 80° bis 110° zueinanderstehen.

Der angegebene Winkelbereich gewährleistet ein besonders ergonomisches Verbinden bzw. Trennen des Anschlussadapters und der Anschlussfläche, wobei ein Abknicken der Fluidleitungen verhindert werden kann. Zudem wird verhindert, dass der Anschlussarm zu weit in den Beladeraum der Reinigungskammer ragt.

Der Anschlussarm kann eine Fluiddurchführung von einer Innenseite der Reinigungskammer zu einer Außenseite der Reinigungskammer aufweisen.

Dadurch dient der Anschlussarm sowohl als Anschlusspunkt für den Anschlussadapter als auch als Fluiddurchführung, um Reinigungsfluid von außerhalb der Reinigungskammer in die Reinigungskammer zu leiten.

Die Anschlussfläche kann im Wesentlichen kreisförmig oder oval ausgebildet sein.

Die Anschlussfläche kann mehrere Reinigungsfluidanschlüsse aufweisen. Jeder Reinigungsfluidanschluss kann zur Verbindung mit einem bestimmten Innenkanal des Medizinprodukts über den Anschlussadapter vorgesehen sein. Die Anschlussfläche kann so eingerichtet sein, dass eine Verbindung mit dem Anschlussadapter nur in einer bestimmten Relativposition zueinander möglich ist.

Dadurch kann ein falsches Anschließen des Anschlussadapters an der Anschlussfläche, d.h. mit verdrehtem Anschlussadapter, verhindert werden und somit immer die richtige Zuordnung der Innenkanäle des Medizinprodukts zu den Reinigungsfluidanschlüssen der Reinigungsvorrichtung gewährleistet werden. Dementsprechend kann eine fehlerhafte Bedienung der Reinigungsvorrichtung verhindert werden.

Die Anzahl und Anordnung der Reinigungsfluidanschlüsse der Anschlussfläche kann derart angepasst sein, um mit einem spezifischen Typ des Anschlussadapters zusammenzupassen.

Durch die angepasste Ausgestaltung der Anschlussfläche kann eine Kompatibilität der Reinigungsvorrichtung mit den üblicherweise verwendeten Anschlussadaptern gewährleistet werden.

Die Anschlussfläche kann durch ein scheibenförmiges Bauteil ausgebildet sein, das den mindestens einen Reinigungsfluidanschluss aufnimmt. Das scheibenförmige Bauteil mit dem Reinigungsfluidanschluss kann in einem Anschlussgehäuse aufgenommen sein. Das Anschlussgehäuse kann abnehmbar an dem Anschlussarm vorgesehen sein.

Durch das abnehmbare Anschlussgehäuse, das die Anschlussfläche mit dem Reinigungsfluidanschluss aufnimmt, können Wartung, Reparatur und beispielsweise eine Kontrolle der Dichtigkeit der Anschlussleitungen innerhalb des Anschlussarms vereinfacht werden.

Die Anschlussfläche kann eingerichtet sein, um mit dem Anschlussadapter eine Steckverbindung zu bilden.

Die Ausführung des Zusammenschließens der Anschlussfläche und des Anschlussadapters als Steckverbindung bieten gute und schnelle Bedienbarkeit.

Die Reinigungsvorrichtung kann mehrere in der Reinigungskammer aufgenommene Siebschalen umfassen, die übereinander angeordnet sind. Für jede der Siebschalen kann mindestens ein Anschlussarm in der Reinigungskammer vorgesehen sein.

Durch die mehreren Siebschalen kann eine Reinigungskapazität der Reinigungsvorrichtung erhöht werden. Dadurch kann bei der Reinigung mehrerer Medizinprodukte Zeit gespart werden und die Effizienz der Reinigungsvorrichtung verbessert werden.

Ein Anschlussarm weist eine Anschlussfläche mit mindestens einem Reinigungsfluidanschluss auf. Der Reinigungsfluidanschluss ist eingerichtet, um mit einer Reinigungsfluidzuführungseinrichtung einer Reinigungsvorrichtung in Verbindung zu stehen. Die Anschlussfläche ist eingerichtet, um mit einem Anschlussadapter, der mindestens eine Reinigungsfluidleitung aufweist, verbindbar zu sein, sodass der Reinigungsfluidanschluss mit der Reinigungsfluidleitung in Verbindung steht, um Reinigungsfluid von der Reinigungsfluidzuführungseinrichtung einem Innenkanal eines Medizinprodukts zur Innenreinigung zuzuführen. Der Anschlussarm umfasst einen ersten Abschnitt, der sich in einer ersten Richtung erstreckt und eingerichtet ist, um an einer Innenwand einer Reinigungskammer der Reinigungsvorrichtung gestützt zu werden, und einen zweiten Abschnitt, der sich in einer zweiten Richtung erstreckt, die bezüglich der ersten Richtung geneigt ist. Die Anschlussfläche ist an dem zweiten Abschnitt vorgesehen.

Weitere Vorteile der vorliegenden Erfindung sind aus der nachfolgenden ausführlichen Beschreibung einer Ausführungsform und den angefügten Zeichnungen ersichtlich.

### Kurze Beschreibung der Zeichnungen

Figur 1 ist eine von der Vorderseite aus gesehene schematische Schnittansicht einer Reinigungsvorrichtung gemäß einer Ausführungsform der vorliegenden Erfindung.
Figur 2 ist eine schematische Perspektivansicht eines Anschlussarms der in Figur 1 gezeigten Reinigungsvorrichtung.
Figur 3 ist eine schematische Explosionsansicht des in Figur 2 gezeigten Anschlussarms.
Figur 4 ist eine schematische Ansicht einer Seitenwand einer Reinigungskammer der in Figur 1 gezeigten Reinigungsvorrichtung.

### Beschreibung von mindestens einer beispielhaften Ausführungsform der Erfindung

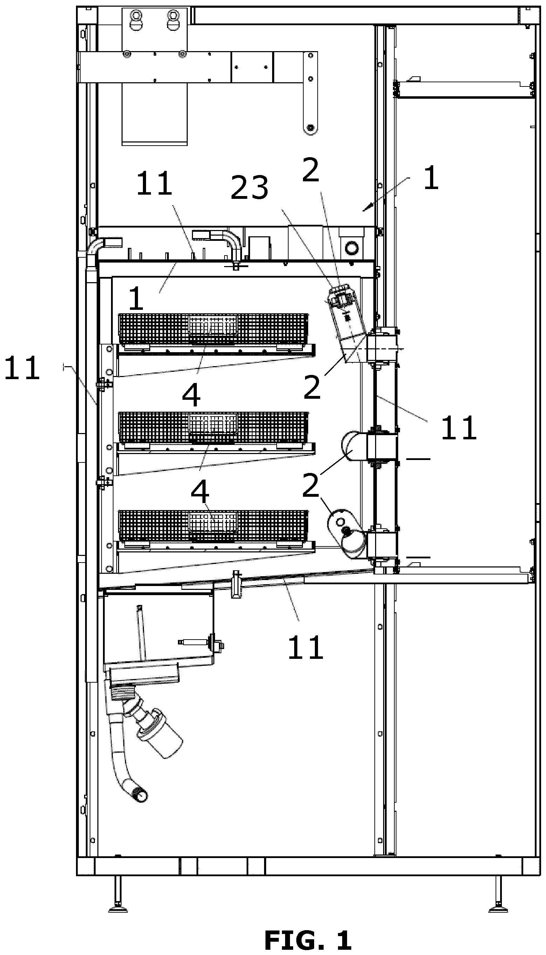

Nachfolgend wird unter Bezugnahme auf Figuren 1 bis 4 eine beispielhafte Ausführungsform der vorliegenden Erfindung beschrieben.

Figur 1 zeigt eine schematische Schnittansicht der Reinigungsvorrichtung von der Vorderseite aus gesehen. Die Reinigungsvorrichtung umfasst eine Reinigungskammer 1. In der vorliegenden Ausführungsform ist die Reinigungskammer 1 als im Wesentlichen rechteckige Kammer ausgebildet, die zwei entgegengesetzt angeordnete Öffnungen zum Be- bzw. Entladen der Reinigungskammer 1 aufweist. Eine erste Öffnung ist an der Vorderseite der in Figur 1 gezeigten Reinigungskammer 1 ausgebildet. Eine zweite Öffnung ist an der Rückseite der Reinigungskammer 1 ausgebildet. In der vorliegenden Ausführungsform sind die erste Öffnung und die zweite Öffnung parallel zu einer Vertikalebene vorgesehen.

Die Reinigungskammer 1 umfasst eine Innenwand 11, die einen im Wesentlichen quaderförmigen Kammerinnenraum umschließt. Die Innenwand 11 umfasst eine obere Wand 111, eine der oberen Wand 111 gegenüberliegende untere Wand 112, eine erste Seitenwand 113 und eine der ersten Seitenwand 113 gegenüberliegende zweite Seitenwand 114. Die Wände 111, 112, 113, 114 können die Innenwand 11 der Reinigungskammer 1 als Einheit ausbilden.

Die untere Wand 112 ist zu einem in der Reinigungskammer 1 vorgesehenen Ablauf hin leicht abfallend ausgebildet, um ein Ablaufen von Flüssigkeiten in der Reinigungskammer 1 zu unterstützen.

Die erste und die zweite Öffnung sind durch geeignet vorgesehene Türen (nicht gezeigt) verschließbar, sodass der Kammerinnenraum gegenüber der Umgebung hermetisch abgedichtet werden kann.

Die Angaben "oben", "unten", "Vorder...", "Rück...", "Seiten...", "horizontal" und "vertikal" beziehen sich auf Positions- und Richtungsangaben bei einer bestimmungsgemäßen Aufstellung der Reinigungsvorrichtung.

Die Reinigungsvorrichtung umfasst eine in der Reinigungskammer 1 angeordnete Halteeinrichtung für mindestens eine Siebschale 4. Die Halteeinrichtung ist in der in Figur 1 gezeigten Ausführungsform derart gestaltet, dass mehrere horizontale Abstellflächen, die vertikal übereinander angeordnet sind, in der Reinigungskammer 1 vorgesehen sind. Die Abstellflächen sind beispielsweise in der Gestalt von Gitterrosten ausgebildet, um sicherzustellen, dass Reinigungsfluid die Abstellflächen möglichst ungehindert durchdringen kann.

Mindestens eine Siebschale 4 kann in der Reinigungskammer 1 aufgenommenen werden. Die Siebschale 4 kann als einfacher Korb gestaltet sein, in den die zu reinigenden Medizinprodukte gelegt werden können. Die Siebschale 4 kann jedoch auch an spezielle zu reinigende Medizinprodukte, beispielsweise Endoskope, angepasst sein, sodass bestimmte Teile der zu reinigenden Medizinprodukte in der Siebschale 4 in einer Position gehalten werden können, die ein möglichst vollständiges Benetzen der Oberflächen der Medizinprodukte mit dem Reinigungsfluid während des Reinigungsvorgangs in der Reinigungsvorrichtung ermöglicht.

Die Siebschale 4 kann für eine Beladung mit den zu reinigenden Medizinprodukten aus der Reinigungskammer 1 genommen werden und nach dem Beladen wieder in die Reinigungskammer 1 eingeführt werden.

Die Reinigungskammer 1 ist des Weiteren so gestaltet, dass mehrere Siebschalen 4 in der Reinigungskammer 1 aufgenommen werden können. In der in Figur 1 gezeigten Ausführungsform können in der Reinigungskammer 1 drei Siebschalen 4 übereinander angeordnet werden, sodass eine oberste, mittlere und unterste Siebschale 4 in der Reinigungskammer 1 aufgenommen werden können. Insbesondere können die Siebschalen 4 auf die durch die Halteeinrichtung ausgebildeten Abstellflächen gestellt werden.

Die Reinigungsvorrichtung umfasst des Weiteren eine oder mehrere Sprüheinrichtungen (nicht gezeigt), die in der Reinigungskammer 1 vorgesehen sind. Die Sprüheinrichtungen dienen dazu, Reinigungsfluid innerhalb der Reinigungskammer 1 so zu verteilen, dass alle Außenflächen der zu reinigenden Gegenstände in der Reinigungskammer 1 sowie alle Wandinnenflächen der Reinigungskammer 1 möglichst gleichmäßig mit dem Reinigungsfluid benetzt werden. Beispielsweise können die Sprüheinrichtungen Sprüharme sein, die sich innerhalb einer horizontalen Ebene drehen.

Des Weiteren umfasst die Reinigungsvorrichtung einen Anschlussarm 2, der in der Reinigungskammer 1 vorgesehen ist.

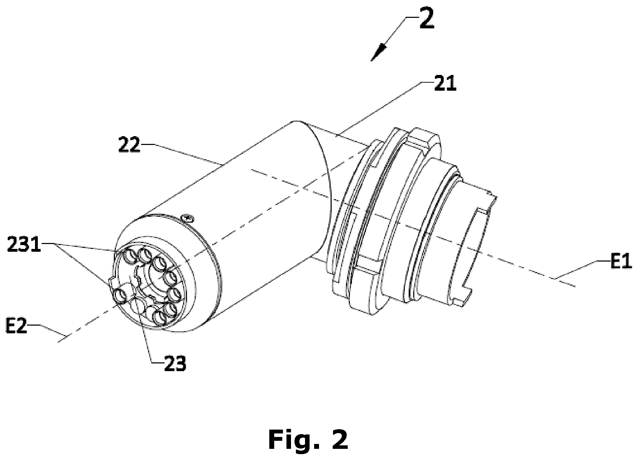

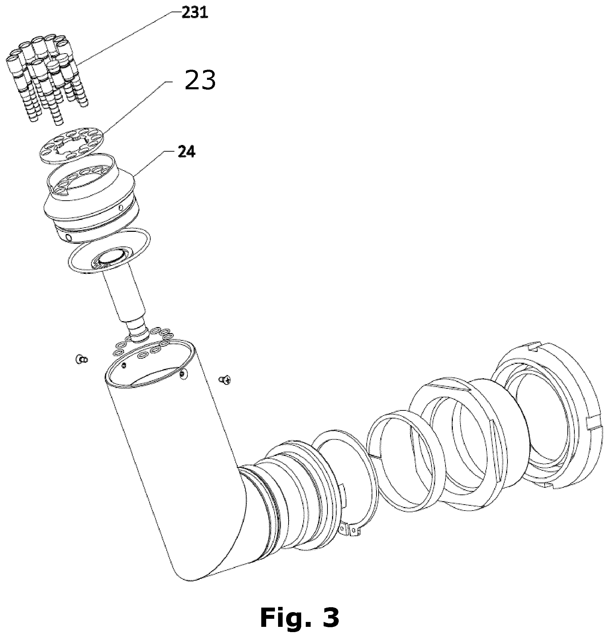

Figur 2 zeigt eine schematische Schnittansicht und Figur 3 eine schematische Explosionsansicht des in der Reinigungskammer 1 angebrachten Anschlussarms 2.

Der Anschlussarm 2 umfasst einen ersten Abschnitt 21, der sich zylinderförmig in einer ersten Richtung E1 erstreckt. Der erste Abschnitt 21 ist schwenkbar an der Innenwand 11 der Reinigungskammer 1 angebracht. Der erste Abschnitt 21 durchdringt die Innenwand 11 der Reinigungskammer 1, sodass ein Ende des ersten Abschnitts 21 außerhalb der Reinigungskammer 1 liegt.

An dem anderen Ende des ersten Abschnitts 21 ist ein zweiter Abschnitt 22 des Anschlussarms 2 angebracht, der sich zylinderförmig in einer zweiten Richtung E2 erstreckt. Die zweite Richtung E2 ist bezüglich der ersten Richtung E1 geneigt. In der vorliegenden Ausführungsform ist die zweite Richtung E2 bezüglich der ersten Richtung E1 derart geneigt, dass der erste Abschnitt 21 und der zweite Abschnitt 22 zueinander in einem Winkel o von ca. 106° stehen. Der Winkel α kann jedoch je nach Anforderung und Platzbedarf innerhalb der Reinigungskammer 1 zwischen 80° und 110° liegen.

Der Anschlussarm 2 umfasst eine Anschlussfläche 23, die an dem zweiten Abschnitt 22 vorgesehen ist. Insbesondere ist die Anschlussfläche 23 an einem Ende des zweiten Abschnitts 22 vorgesehen, das bezüglich des ersten Abschnitts 21 weiter entfernt ist. Die Anschlussfläche 23 weist mehrere Reinigungsfluidanschlüsse 231 auf. Wie in Figur 3 gezeigt ist, wird die Anschlussfläche 23 durch ein im Wesentlichen scheibenförmiges Bauteil gebildet, in dem entsprechende Aussparungen für die Reinigungsfluidanschlüsse 231 vorgesehen sind.

In der vorliegenden Ausführungsform ist die Anschlussfläche 23 bezüglich der zweiten Richtung E2 rechtwinklig ausgebildet. Die Anschlussfläche 23 kann jedoch auch bezüglich der zweiten Richtung E2 geneigt ausgeführt werden. Beispielsweise können die Anschlussfläche 23 und die zweite Richtung E2 je nach Anforderung und Platzbedarf zueinander in einem Winkel von 25° bis 90° stehen. Zudem kann die Anschlussfläche 23 wie in der vorliegenden Ausführungsform kreisförmig oder alternativ auch oval ausgebildet werden.

Die Anschlussfläche 23 ist mit den Reinigungsfluidanschlüssen 231 in einem Anschlussgehäuse 24 aufgenommen. Das Anschlussgehäuse 24 ist abnehmbar an dem Anschlussarm 2 montiert. Genauer gesagt ist das Anschlussgehäuse 24 an dem zweiten Abschnitt 22 des Anschlussarms 2 abnehmbar vorgesehen. Jeder der Reinigungsfluidanschlüsse 231 steht über eine entsprechend vorgesehene Anschlussleitung (nicht gezeigt), die im Inneren des Anschlussarms 2 in Richtung einer Außenseite der Reinigungskammer 1 führt, mit einer Reinigungsfluidzuführungseinrichtung der Reinigungsvorrichtung in Verbindung. Die Reinigungsfluidzuführungseinrichtung kann beispielsweise eine Fluidpumpe sein, die das Reinigungsfluid fördert.

Der erste Abschnitt 21 und der zweite Abschnitt 22 können als Einheit ausgebildet sein. Die jeweiligen Abmessungen des ersten Abschnitts 21 und des zweiten Abschnitts 22 in der ersten bzw. der zweiten Richtung E1, E2 können je nach Anforderung und Platzbedarf in der Reinigungskammer 1 gewählt werden.

In der vorliegenden Ausführungsform umschließen die zylinderförmigen Abschnitte 21, 22 des Anschlussarms 2 einen radial innenliegenden Hohlraum. Dieser Hohlraum dient zur Führung der Anschlussleitungen, die die Reinigungsfluidanschlüsse 231 mit der Reinigungsfluidzuführungseinrichtung verbinden. Somit bildet der Anschlussarm 2 eine Fluiddurchführung von der Innenseite zu der Außenseite der Reinigungskammer 1.

Die Reinigungsfluidanschlüsse 231 sind in der Anschlussfläche 23 derart vorgesehen, dass ein Anschlussadapter (nicht gezeigt) an der Anschlussfläche 23 angeschlossen werden kann, der jeweils eine Fluidverbindung zwischen einem der Reinigungsfluidanschlüsse 231 und einem an dem Anschlussadapter angebrachten Schlauch (Reinigungsfluidleitung) herstellt. Die Schläuche des Anschlussadapters sind wiederum über spezielle Konnektoren mit den zu reinigenden Innenräumen des Medizinprodukts (z.B. Innenkanäle eines Endoskops) verbunden.

Das Zusammenschließen des Anschlussadapters und der Anschlussfläche 23 und das damit einhergehende Verbinden der Reinigungsfluidanschlüsse 231 mit je einem Schlauch des Anschlussadapters kann beispielsweise über eine geeignete Steckverbindung erfolgen. Die Steckverbindung kann beispielsweise derart ausgeführt werden, dass ein Zusammenschließen des Anschlussadapters und der Anschlussfläche 23 nur in einer bestimmten Relativposition zueinander möglich ist, um eine eindeutige Zuordnung der Reinigungsfluidanschlüsse 231 zu den Schläuchen des Anschlussadapters sicherzustellen.

Der Anschlussarm 2 umfasst eine Lagerung, die ein Schwenken des Anschlussarms 2 um die erste Richtung E1 ermöglicht. Wie in den Figuren 2 und 3 gezeigt ist, ist eine entsprechende Lagerung an einem Außenumfang des ersten Abschnitts 21 vorgesehen. Wie oben erwähnt, ist der erste Abschnitt 21 mittels der Lagerung an der Innenwand 11 der Reinigungskammer 1 schwenkbar angebracht. In der vorliegenden Ausführungsform ist der Anschlussarm 2 an der zweiten Seitenwand 114 schwenkbar angebracht.

Die Lagerung des Anschlussarms 2 ist mit einer entsprechenden Dichtungseinrichtung versehen, sodass die erforderliche Abdichtung der Reinigungskammer 1 während des Reinigungsvorgangs gewährleistet werden kann.

In der vorliegenden Ausführungsform sind mehrere Anschlussarme 2 in der Reinigungskammer 1 angeordnet. Ein erster Anschlussarm 2 ist an der zweiten Seitenwand 114 angeordnet. Der erste Anschlussarm 2 ist im Wesentlichen mittig zwischen der ersten und der zweiten Öffnung angeordnet (d.h. eine Position des Anschlussarms 2 bezüglich einer Horizontalrichtung bei bestimmungsgemäßer Aufstellung der Reinigungsvorrichtung). Der erste Anschlussarm 2 befindet sich auf einer Höhe, die im Wesentlichen einer Position einer Unterseite der obersten Siebschale 4 entspricht (d.h. eine Position des Anschlussarms 2 bezüglich einer Vertikalrichtung bei bestimmungsgemäßer Aufstellung der Reinigungsvorrichtung).

Ein zweiter Anschlussarm 2 ist an einer der beiden Seitenwände 113, 114 angeordnet. In der vorliegenden Ausführungsform sind der erste, zweite und ein dritter Anschlussarm 2 an derselben Seitenwand 114 angeordnet. Die Anschlussarme 2 können jedoch auch an verschiedenen Seitenwänden angeordnet werden.

Der zweite Anschlussarm 2 ist im Wesentlichen mittig zwischen der ersten und der zweiten Öffnung angeordnet. Der zweite Anschlussarm 2 befindet sich auf einer Höhe, die im Wesentlichen einer Position einer Unterseite der mittleren Siebschale 4 entspricht.

Der dritte Anschlussarm 2 ist an der zweiten Seitenwand 114 im Wesentlichen mittig zwischen der ersten und der zweiten Öffnung angeordnet. Der dritte Anschlussarm 2 befindet sich auf einer Höhe, die im Wesentlichen einer Position einer Unterseite der untersten Siebschale 4 entspricht.

Somit ist für jede Siebeschale 4, die in der Reinigungskammer 1 aufgenommen werden kann, ein Anschlussarm 2 vorgesehen. Folglich können in der vorliegenden Ausführungsform beispielsweise drei Endoskope gleichzeitig in der Reinigungsvorrichtung wiederaufbereitet werden.

Weder die Anzahl der Siebschalen 4, die in der Reinigungskammer 1 aufgenommen werden können, noch die Anzahl der in der Reinigungskammer 1 angeordneten Anschlussarme 2 sind beschränkt und können gemäß einer Größe der Reinigungskammer 1 angepasst werden.

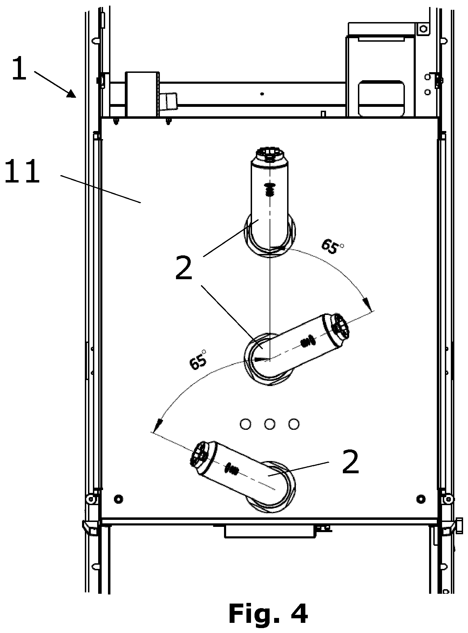

Figur 4 zeigt eine Ansicht der zweiten Seitenwand 114 der Reinigungskammer 1 von der Mitte der Reinigungskammer 1 aus gesehen.

Wie oben beschrieben ist, sind drei Anschlussarme 2 auf verschiedenen Höhen angebracht. Bezüglich einer horizontalen Position sind die Anschlussarme 2 zwischen der ersten Öffnung (rechte Seite in Figur 4), die beispielsweise eine Beladeseite der Reinigungsvorrichtung definiert, und der zweiten Öffnung (linke Seite in Figur 4), die beispielsweise eine Entladeseite der Reinigungsvorrichtung definiert, mittig angeordnet.

Die drei Anschlussarme 2 sind zur Erläuterung eines Schwenkwinkels des Anschlussarms 2 in unterschiedlichen Positionen bezüglich des Schwenkwinkels dargestellt.

Der oben angeordnete Anschlussarm 2 befindet sich bezüglich des Schwenkwinkels an einer neutralen Position. Das heißt, die Anschlussfläche 23 ist bezüglich der Innenwand 11 weder in Richtung der ersten Öffnung noch in Richtung der zweiten Öffnung geneigt. Genauer gesagt ist die Anschlussfläche 23 weder der ersten Öffnung noch der zweiten Öffnung zugewandt. In der neutralen Position liegen die erste Richtung E1 und die zweite Richtung E2 in einer Vertikalebene.

Der bezüglich der vertikalen Anordnung in der Mitte gelegene Anschlussarm 2 befindet sich bezüglich des Schwenkwinkels an einer ersten Position, die einem maximalen Schwenkwinkel des Anschlussarms 2 in Richtung der ersten Öffnung entspricht. Wenn sich der Anschlussarm 2 in der ersten Position befindet, ist die Anschlussfläche 23 bezüglich der Innenwand 11 in Richtung der ersten Öffnung geneigt. Genauer gesagt, die Anschlussfläche 23 ist der ersten Öffnung zugewandt.

Der unten angeordnete Anschlussarm 2 befindet sich bezüglich des Schwenkwinkels an einer zweiten Position, die einem maximalen Schwenkwinkel des Anschlussarms 2 in Richtung der zweiten Öffnung entspricht. Wenn sich der Anschlussarm 2 in der zweiten Position befindet, ist die Anschlussfläche 23 bezüglich der Innenwand 11 in Richtung der zweiten Öffnung geneigt. Genauer gesagt, die Anschlussfläche 23 ist der zweiten Öffnung zugewandt.

In der vorliegenden Ausführungsform ist der maximale Schwenkwinkel des Anschlussarms 2 von der neutralen Position in die erste Position gleich dem maximalen Schwenkwinkel des Anschlussarms 2 von der neutralen Position in die zweite Position. Das heißt, ein Schwenkbereich des Anschlussarms 2 ist bezüglich einer Vertikalebene symmetrisch vorgesehen. Wie in Figur 4 gezeigt ist, sind in der vorliegenden Ausführungsform die erste Öffnung und die zweite Öffnung parallel zu einer Vertikalebene vorgesehen.

In der vorliegenden Ausführungsform ist der Anschlussarm 2 von der neutralen Position aus maximal um 65° in Richtung der ersten bzw. der zweiten Öffnung schwenkbar. Somit ergibt sich ein maximaler Schwenkbereich des Anschlussarms 2 von 130°. Der maximale Schwenkbereich kann je nach Anforderung und Platzbedarf angepasst werden und kann zwischen 90° und 170° liegen.

In der oben beschriebenen Ausführungsform ist die Anschlussfläche 23 an dem zweiten Abschnitt 22 des Anschlussarms 2 vorgesehen. Die vorliegende Erfindung ist aber darauf nicht beschränkt. Der Anschlussarm kann beispielsweise nur einen zylinderförmigen Abschnitt aufweisen und die Anschlussfläche kann an dem einen Abschnitt vorgesehen sein. Die Anschlussfläche kann bezüglich einer Richtung, in die sich der eine Abschnitt des Anschlussarm erstreckt, geneigt sein, um je nach Schwenkwinkel des Anschlussarms der ersten oder der zweiten Öffnung der Reinigungskammer 1 zugewandt zu sein.

Die obige Beschreibung ist nicht erschöpfend und die vorliegende Erfindung ist nicht auf die oben genannte(n) Ausführungsform(en) beschränkt. Der Fachmann wird erkennen, dass innerhalb des Umfangs der vorliegenden Erfindung verschiedene Abwandlungen und Kombinationen der in der/den obigen Ausführungsform(en) enthaltenen Merkmale möglich sind. Daher sollte der Umfang der vorliegenden Erfindung durch die beigefügten Ansprüche bestimmt werden.

## Patentansprüche

1. Reinigungsvorrichtung zum Reinigen und Desinfizieren von wiederaufbereitbaren Medizinprodukten, insbesondere Endoskopen, mit
einer Reinigungskammer (1), die mindestens eine verschließbare Öffnung zum Be- und/oder Entladen der Reinigungskammer (1) aufweist, und
einem Anschlussarm (2), der in der Reinigungskammer (1) vorgesehen ist, wobei
der Anschlussarm (2) eine Anschlussfläche (23) mit mindestens einem Reinigungsfluidanschluss (231) aufweist,
der Reinigungsfluidanschluss (231) mit einer Reinigungsfluidzuführungseinrichtung der Reinigungsvorrichtung in Verbindung steht,
die Anschlussfläche (23) eingerichtet ist, um mit einem Anschlussadapter, der mindestens eine Reinigungsfluidleitung aufweist, verbindbar zu sein, so dass der Reinigungsfluidanschluss mit der Reinigungsfluidleitung in Verbindung steht, um Reinigungsfluid von der Reinigungsfluidzuführungseinrichtung einem Innenkanal des Medizinprodukts zur Innenreinigung zuzuführen, und
die Anschlussfläche (23) bezüglich einer Innenwand (11) der Reinigungskammer (1) in Richtung der Öffnung der Reinigungskammer (1) geneigt ist.

2. Reinigungsvorrichtung gemäß Anspruch 1, wobei
die Reinigungskammer (1) eine erste verschließbare Öffnung zum Beladen und eine der ersten Öffnung entgegengesetzte zweite verschließbare Öffnung zum Entladen aufweist,
der Anschlussarm (2) schwenkbar in der Reinigungskammer (1) vorgesehen ist, sodass der Anschlussarm (2) zwischen einer ersten Position, in der die Anschlussfläche (23) bezüglich der Innenwand (11) in Richtung der ersten Öffnung geneigt ist, und einer zweiten Position schwenkbar ist, in der die Anschlussfläche (23) bezüglich der Innenwand (11) in Richtung der zweiten Öffnung geneigt ist.

3. Reinigungsvorrichtung gemäß Anspruch 1 oder 2, wobei
der Anschlussarm (2) einen ersten Abschnitt (21), der sich in einer ersten Richtung (E1) erstreckt und an der Innenwand (11) der Reinigungskammer (1) gestützt ist, und einen zweiten Abschnitt (22) umfasst, der sich in einer zweiten Richtung (E2) erstreckt, die bezüglich der ersten Richtung (E1) geneigt ist, und
die Anschlussfläche (23) an dem zweiten Abschnitt (22) vorgesehen ist.

4. Reinigungsvorrichtung gemäß Anspruch 2 oder 3, wobei
der erste Abschnitt (21) an der Innenwand (11) der Reinigungskammer (1) um die erste Richtung (E1) herum schwenkbar gestützt ist.

5. Reinigungsvorrichtung gemäß einem der Ansprüche 2 bis 4, wobei
der Schwenkbereich des Anschlussarms (2) bezüglich einer Ebene, die parallel zu der ersten und der zweiten Öffnung verläuft, symmetrisch vorgesehen ist.

6. Reinigungsvorrichtung gemäß einem der Ansprüche 3 bis 5, wobei
der erste Abschnitt (21) an der Innenwand (11) im Wesentlichen mittig zwischen der ersten Öffnung und der zweiten Öffnung vorgesehen ist.

7. Reinigungsvorrichtung gemäß einem der Ansprüche 2 bis 6, wobei
die Reinigungskammer (1) eine obere Innenwand (111), eine untere Innenwand (112), sowie eine erste und eine zweite Seiteninnenwand (113, 114) aufweist,
der erste Abschnitt (21) an einer von der ersten und der zweiten Seiteninnenwand (113, 114) gestützt ist und sich parallel zu der oberen Innenwand (111, 112) sowie senkrecht zu der Seitenwand (113) erstreckt.

8. Reinigungsvorrichtung gemäß einem der Ansprüche 2 bis 7, wobei
die zweite Richtung (E2), in die sich der zweite Abschnitt (22) erstreckt, und die erste Richtung (E1), in die sich der erste Abschnitt (21) erstreckt, in einem Winkel (α) von 80° bis 110° zueinander stehen.

9. Reinigungsvorrichtung gemäß einem der vorhergehenden Ansprüche, wobei
der Anschlussarm (2) eine Fluiddurchführung von einer Innenseite der Reinigungskammer (1) zu einer Außenseite der Reinigungskammer (1) aufweist.

10. Reinigungsvorrichtung gemäß einem der vorhergehenden Ansprüche, wobei
die Anschlussfläche (23) mehrere Reinigungsfluidanschlüsse (231) aufweist,
jeder Reinigungsfluidanschluss (231) zur Verbindung mit einem bestimmten Innenkanal des Medizinprodukts über den Anschlussadapter vorgesehen ist, und
die Anschlussfläche (23) so eingerichtet ist, dass eine Verbindung mit dem Anschlussadapter nur in einer bestimmten Relativposition zueinander möglich ist.

11. Reinigungsvorrichtung gemäß einem der vorhergehenden Ansprüche, wobei
die Anzahl und Anordnung der Reinigungsfluidanschlüsse (231) der Anschlussfläche (23) derart angepasst sind, um mit einem spezifischen Typ des Anschlussadapters zusammenzupassen.

12. Reinigungsvorrichtung gemäß einem der vorhergehenden Ansprüche, wobei
die Anschlussfläche (23) durch ein scheibenförmiges Bauteil (232) ausgebildet ist, das den mindestens einen Reinigungsfluidanschluss (231) aufnimmt, und
das scheibenförmige Bauteil (232) mit dem Reinigungsfluidanschluss (231) in einem Anschlussgehäuse (24) aufgenommen ist,
das Anschlussgehäuse (24) abnehmbar an dem Anschlussarm (2) vorgesehen ist.

13. Reinigungsvorrichtung gemäß einem der vorhergehenden Ansprüche, wobei
die Anschlussfläche (23) eingerichtet ist, um mit dem Anschlussadapter eine Steckverbindung zu bilden.

14. Reinigungsvorrichtung gemäß einem der vorhergehenden Ansprüche, wobei
die Reinigungsvorrichtung mehrere in der Reinigungskammer (1) aufgenommene Siebschalen (4) umfasst, die übereinander angeordnet sind, und
für jede der Siebschalen (4) mindestens ein Anschlussarm (2) in der Reinigungskammer (1) vorgesehen ist.

15. Anschlussarm (2) für eine Reinigungsvorrichtung zum Reinigen von wiederaufbereitbaren Medizinprodukten, insbesondere Endoskopen, wobei
der Anschlussarm (2) eine Anschlussfläche (23) mit mindestens einem Reinigungsfluidanschluss (231) aufweist, wobei der Reinigungsfluidanschluss (231) eingerichtet ist, um mit einer Reinigungsfluidzuführungseinrichtung der Reinigungsvorrichtung in Verbindung zu stehen,
die Anschlussfläche (23) eingerichtet ist, um mit einem Anschlussadapter, der mindestens eine Reinigungsfluidleitung aufweist, verbindbar zu sein, so dass der Reinigungsfluidanschluss mit der Reinigungsfluidleitung in Verbindung steht, um Reinigungsfluid von der Reinigungsfluidzuführungseinrichtung einem Innenkanal des Medizinprodukts zur Innenreinigung zuzuführen,
der Anschlussarm (2) einen ersten Abschnitt (21), der sich in einer ersten Richtung (E1) erstreckt und eingerichtet ist, um an einer Innenwand (11) einer Reinigungskammer (1) der Reinigungsvorrichtung gestützt zu werden, und einen zweiten Abschnitt (22) umfasst, der sich in einer zweiten Richtung (E2) erstreckt, die bezüglich der ersten Richtung (E1) geneigt ist, und
die Anschlussfläche (23) an dem zweiten Abschnitt (22) vorgesehen ist.
